Europäisches Patentamt

European Patent Office

Office européen des brevets

(11) Publication number: **0 471 392 A2**

# EUROPEAN PATENT APPLICATION

(21) Application number: **91201722.5**

(22) Date of filing: **04.07.91**

(51) Int. Cl.5: **A61K 7/32**, A61K 7/36,
A61K 7/48

(30) Priority: **13.07.90 GB 9015502**

(43) Date of publication of application:
**19.02.92 Bulletin 92/08**

(84) Designated Contracting States:
**AT BE CH DE DK ES FR GB GR IT LI NL SE**

(71) Applicant: **UNILEVER NV**
**Burgemeester s'Jacobplein 1 P.O. Box 760**
**NL-3000 DK Rotterdam(NL)**
(84) **BE CH DE DK ES FR GR IT LI NL SE AT**

(71) Applicant: **UNILEVER PLC**
**Unilever House Blackfriars**
**London EC4P 4BO(GB)**
(84) **GB**

(72) Inventor: **Park, Andrew Campbell, Unilever**
**Research**
**Port Sunlight Laboratory, Ouarry Road East**
**Bebington, Wirral, Merseyside L63 3JW(GB)**

(74) Representative: **van Gent, Jan Paulus et al**
**Unilever N.V., Patent Division Postbus 137**
**NL-3130 AC Vlaardingen(NL)**

(54) **Deodorant composition.**

(57) A propellant-free deodorant composition suitable for topical application to human skin, which comprises:
i an effective amount of a deodorant chosen from salts or oxides of zinc or magnesium, which are sparingly soluble in water;
ii a cellulosic polymer having a water absorbing capacity of at least 1; and
iii a volatile silicone.

EP 0 471 392 A2

## FIELD OF THE INVENTION

The invention relates to deodorant compositions suitable for topical application to human skin, particularly liquid compositions suitable for use with a roll-on dispenser, or solid compositions suitable for use as a cosmetic stick together with a stick holder.

## BACKGROUND TO THE INVENTION

The deodorant and anti-perspirant market is dominated with products based on aluminium or zirconium salts which are intended to prevent, or at least control, perspiration at the skin surface, particularly on the underarm, while simultaneously providing a perceived degree of deodorancy.

It has also been proposed in US 4,605,553 (Passalacqua) to employ aluminium chlorhydrate in an antiperspirant dry pressed powder stick which otherwise also contains zinc stearate, magnesium carbonate and alpha cellulose. The cellulose ingredient is said to enhance flowability of the powder blend when formed into a stick, and it also adds to the cosmetic feel on the skin and can assist in binding the powdered material together to give the stick strength.

Certain doubts as to the safety in use of aluminium salts have recently stimulated a search for alternative antiperspirant active materials. As a result of this need, we have now developed a totally new approach to the control of perspiration at the skin surface, while maintaining deodorancy, by combining a moisture-absorbent cellulosic polymer, which literally mops up perspiration as it appears at the skin's surface, with a special salt, which not only performs a deodorancy function, but which does not adversely affect the efficacy of the moisture-absorbent polymer.

## DEFINITION OF THE INVENTION

Accordingly, the invention provides a propellant-free deodorant composition suitable for topical application to human skin, which comprises:

i. an effective amount of a deodorant chosen from salts or oxides of zinc or magnesium, which are sparingly soluble in water;

ii. a cellulosic polymer having a water absorbing capacity of at least 1; and

iii. a volatile silicone.

## DISCLOSURE OF THE INVENTION

The Deodorant

The composition according to the invention comprises a deodorant which is chosen from salts or oxides of zinc or magnesium which are insoluble or only sparingly soluble in water.

Examples of suitable salts or oxides are:

zinc oxide
zinc carbonate
zinc citrate
zinc laurate
zinc oleate
zinc orthophosphate
zinc stearate
zinc silicate
zinc tartrate
magnesium oxide
magnesium carbonate
magnesium fluoride
magnesium hydroxide
magnesium laurate
magnesium myristate
magnesium oleate
magnesium palmitate
magnesium stearate
magnesium orthophosphate

magnesium pyrophosphate
magnesium silicate

The amount of deodorant present in the composition according to the invention is from 0.5 to 20%, preferably from 1 to 15% by weight of the composition.

## The Cellulosic Polymer

The composition according to the invention also comprises a cellulosic polymer having a water absorbing capacity of at least 1.

By "water absorbing capacity" we mean that the polymer has the ability to absorb an amount of water at least equal to its own weight.

Preferably the cellulosic polymer will have a water absorbing capacity of at least 5, most preferably of at least 10 or even higher.

Particularly preferred examples of cellulosic polymers are cross-linked carboxymethylcelluloses, such as AKUCELL, available from AKZO, and AQUALON available from HERCULES.

The amount of cellulosic polymer present in the composition according to the invention is from 1 to 50%, preferably from 5 to 30% by weight of the composition.

## The Volatile Silicone

The composition according to the invention also comprises a linear or cyclic volatile silicone.

Examples of volatile silicones include polydimethyl cyclosiloxane, having a viscosity of less than $5mm^2s^{-1}$, examples of which are DOW CORNING 344 Fluid (tetramer) and DOW CORNING 345 Fluid (pentamer), and volatile hexamethyldisiloxane having a viscosity of not more than $0.65mm^2s^{-1}$, for example DOW CORNING 200 Fluid ($0.65mm^2s^{-1}$).

The preferred volatile silicones are the cyclic forms.

The amount of volatile silicone present in the composition according to the invention is from 1 to 90%, preferably from 5 to 70% by weight of the composition.

## Other Ingredients

The composition according to the invention can comprise other ingredients, in addition to those already identified, depending on the nature and form of the finished product.

Examples of other ingredients which can optionally be present in the composition according to the invention include:

non-volatile silicones, such as a polydimethylsiloxane having a viscosity in excess of $5mm^2s^{-1}$, for example, from 50 to $1000mm^2s^{-1}$, such as DOW CORNING 200 Fluids (standard viscosities $50-1000mm^2s^{-1}$);

thickeners, such as clays, for example Bentone 38; silica, for example Aerosil 200;

skin feel improves, such as talc and finely divided polyethylene, an example of which is ACUMIST B18;

cosmetically acceptable vehicles, such as anhydrous ethanol and other emollients;

gelling agents, such as stearyl alcohol or waxes, for example Castor wax;

perfumes;

preservatives; and

other cosmetic adjuncts conventionally employed in stick, roll-on and spray deodorant products.

The ingredients other than the deodorant, cellulosic polymer and volatile silicone which can optionally be present in the composition according to the invention can conveniently form the balance of the composition, and accordingly can form up to 90%, preferably from 10 to 80% by weight of the composition.

## Product Form

The composition according to the invention can take the form of liquid or solid products, each of which is suited to or adapted for topical application to human skin. One convenient form of the composition according to the invention is a solid stick, usually contained in a suitable holder or dispenser to enable it to be applied to the area of the skin, particularly the underarm, where control of perspiration and deodorancy is required.

Another form of the composition of the invention is a lotion suitable for inclusion in a roll-on dispenser, fitted with a ball valve, to enable the product to be rolled onto the skin in a manner which is conventional in

EP 0 471 392 A2
the art. A further example of a composition according to the invention is the liquid composition for dispensing via a finger operated pump-spray or a hand operated squeeze spray to provide for delivery to the skin of a finely divided spray or aerosol, without the use of environmentally unfriendly propellant gases to deliver it.

Use of the composition

The invention also provides for the use of a propellant-free deodorant composition, in accordance with the invention, as herein defined, in perspiration and body malodour control, following topical application to human skin.

A preferred embodiment of the invention provides for the use of a propellant-free deodorant composition comprising

i. from 0.5 to 20% by weight of zinc carbonate;

ii. from 1 to 50% by weight of cross-linked carboxymethyl cellulose, having a water absorbing capacity of at least 1; and

iii. from 1 to 90% by weight of linear or cyclic volatile silicone;

in controlling perspiration and body malodour following topical application to the skin, particularly the underarm, of the composition according to the invention.

Examples

The invention is further illustrated by the following examples.

Example 1

This examples illustrates a deodorant stick product according to the invention. The stick had the following formulation:

|  | % w/w |
| --- | --- |
| Volatile silicone - DOW CORNING 345 | 61.0 |
| Akucell | 10.0 |
| Zinc carbonate | 6.0 |
| Castor wax | 4.0 |
| Stearyl alcohol | 16.0 |
| PEG 400 distearate | 2.0 |
| Perfume | 1.0 |

Example 2

This example illustrates a deodorant roll-on product according to the invention. The roll-on lotion, for use in a roll-ball applicator, had the following formulation:

|  | % w/w |
| --- | --- |
| Volatile silicone - DOW CORNING 344 + 345 | 59.95 |
| Akucell | 10.0 |
| Zinc carbonate | 8.0 |
| Acumist B18* | 7.0 |
| Bentone 38 | 1.0 |
| Aerosil 200 | 0.55 |
| Silicone 200 fluid $50mm^2s^{-1}$ | 12.0 |
| Ethanol, anhydrous | 0.5 |
| Perfume | 1.0 |

* Finely divided polyethylene powder - particle size 18$\mu$m.

4

Example 3

This example illustrates a deodorant lotion suitable for dispensing from a hand operated squeeze spray device which is operated without environmentally unfriendly gaseous propellants.

|  | % w/w |
|---|---|
| Volatile silicone - DOW CORNING | 12.0 |
| Akucell | 10.0 |
| Zinc carbonate | 2.0 |
| Ethanol, anhydrous | 72.5 |
| Bentone 38 | 2.5 |
| Perfume | 1.0 |

**Claims**

1. A propellant-free deodorant composition suitable for topical application to human skin, which comprises:
   i. an effective amount of a deodorant chosen from salts or oxides of zinc or magnesium, which are sparingly soluble in water;
   ii. a cellulosic polymer having a water absorbing capacity of at least 1; and
   iii. a volatile silicone.

2. A composition according to claim 1, in which the zinc salt is zinc carbonate.

3. A composition according to claim 1 or 2, in which the deodorant forms from 1 to 20% by weight of the composition.

4. A composition according to any of claims 1, 2 or 3, in which the cellulosic polymer has a water absorbing capacity of at least 10.

5. A composition according to any preceding claim, in which the cellulosic polymer is a cross-linked carboxymethyl cellulose.

6. A composition according to any preceding claim, in which the cellulosic polymer forms from 1 to 50% by weight of the composition.

7. A composition according to any preceding claim, in which the volatile silicone is a linear or cyclic polydimethyl siloxane.

8. A compositin according to any preceding claim, in which the volatile silicone form from 1 to 90% by weight of the composition

9. A propellant-free deodorant composition suitable for topical application to human skin which comprises:
   i. from 1 to 20% by weight of zinc carbonate;
   ii. from 1 to 50% by weight of cross-linked carboxymethyl cellulose, having a water absorbing capacity of at least 1; and
   iii. from 1 to 90% by weight of a linear or cyclic volatile silicone.

10. The use of a propellant-free deodorant composition comprising:
    i. an effective amount of a deodorant chosen from salts or oxides of zinc or magnesium, which are sparingly soluble in water;
    ii. a cellulosic polymer having a water absorbent capacity of at least 1; and
    iii. a volatile silicone;
    for the control of perspiration and reduction of body malodour following topical application to human skin.